# EUROPEAN PATENT APPLICATION

(11) **EP 3 957 281 A1**
(43) Date of publication of application: **23.02.2022**
(21) Application number: 19925448.3
(22) Date of filing: 07.08.2019
(51) Int. Cl.: A61F 2/844, A61B 17/12, A61L 31/02, B21F 45/00, A61F 2/82

(54) **CEREBRAL ANEURYSM STENT AND MANUFACTURING METHOD THEREFOR**

(30) Priority: 16.04.2019 KR 20190044437
(71) Applicant: S&G Biotech, Inc., Yongin-si, Gyeonggi-do 17023 (KR)
(72) Inventor: KANG, Sung Kwon, Yongin-si, Gyeonggi-do 16876 (KR)
(74) Representative: Shearman, James Ward
(86) International application number: PCT/KR2019/009857
(87) International publication number: WO 2020/213791

(57) **Abstract**

A cerebral aneurysm stent (1) of the present invention comprises a hollow tubular main body (10) and a coil support (20) integrally formed with the main body (10) at one end of the main body (10) in the longitudinal direction, wherein the coil support (20) comprises a closed structure (21) with respect to the radially inner side of the cerebral aneurysm stent (1), the closed structure (21) being located at an outer end portion of the cerebral aneurysm stent (1) in the longitudinal direction.

## Description

### Technical Field

The present invention relates to a cerebral aneurysm stent for cerebral aneurysm embolization and a method for manufacturing the same, and more particularly, to a cerebral aneurysm stent for stent-assisted coil cerebral aneurysm embolization and a method for manufacturing the same, which uses a stent to prevent a coil inserted into a cerebral aneurysm formed in a branching vessel area.

### Background Art

A cerebral aneurysm is a vascular disease in which the blood vessel walls in the brain have microscopic cracks and abnormally swell. The exact cause is not yet known. In the past, cerebral aneurysms were known as resulting from innate factors, such as weak vessel walls. However, recently believed is that cerebral aneurysms mainly come from acquired factors, such as stress, vasculitis, and damage to vessel walls due to injury.

These cerebral aneurysms usually rupture without any symptoms and are expressed as cerebral hemorrhage, leading to death or very serious sequelae. This is common for ones in their forties to sixties. Further, the meninges are stimulated by bleeding, which may cause vomiting, nausea, severe headache, and stiff neck. In severe cases, pressure within the skull increases, resulting in loss of consciousness or coma.

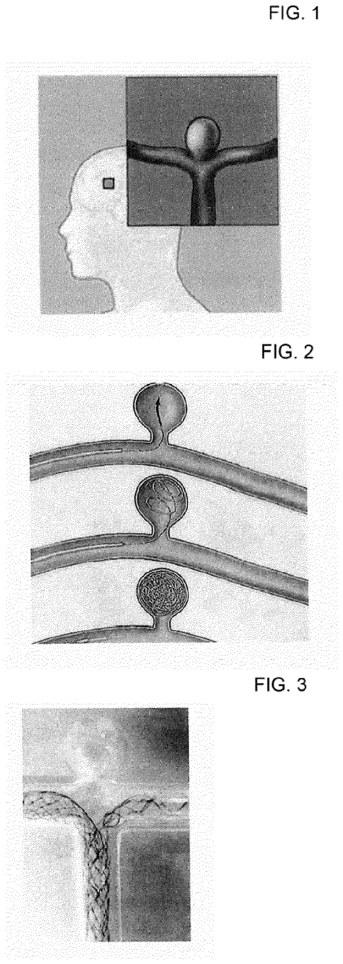

Further, as shown in FIG. 1, cerebral aneurysms mainly occur in the T-shaped branching vessels of the cerebral blood vessels.

As a treatment method for such a cerebral aneurysm, cerebral aneurysm clipping and coil embolization of aneurysm are widely known.

First, in cerebral aneurysm clipping, a piece of the skull is removed, the cerebral aneurysm positioned between the brain tissues is secured, and the origin is clipped with a small clip. However, if complete clipping is impossible due to a bad position or shape, surgery is performed, with the remaining area covered with special gauze to serve as a buffer for possible bleeding.

As shown in Fig. 2, in cerebral aneurysm coil embolization, a micro-conduit is inserted into the cerebral aneurysm without removing some cranial fragments to block only the cerebral aneurysm without invading normal blood vessels. This is a procedure that prevents blood flow from entering the cerebral aneurysm by filling the cerebral aneurysm with a coil of the same embolic material.

Cerebral aneurysm coil embolization gains popularity because it does not require removal of a skull fragment. However, in the case of a large-diameter cerebral aneurysm, the coil may escape off the cerebral aneurysm, and the cerebral aneurysm may recur after the procedure.

To address these issues, there is a known technique for inserting a stent to prevent the coil from the brain aneurysm. This technique is also called stent-assisted coil embolization. As shown in FIG. 3, two stents are inserted into branching vessels in a Y-shape to support the coil not to escape from the cerebral aneurysm.

However, in the conventional stent-assisted coil embolization, two stents are overlapped in a Y-shape for a cerebral aneurysm occurring in branching vessels, so the procedure is complicated and requires a high-level surgical technique for clinicians. there is also a problem of an increase in the risk of complications due to the use of two stents. Further, there is a problem that the overlap of the two stents causes expansion difficult.

### Detailed Description of the Invention

### Technical Problems

In view of the foregoing issues, the present invention aims to address the problems with the conventional cerebral aneurysm stent and provide a cerebral aneurysm stent applicable to T-shaped branching vessels and a method for manufacturing the same.

### Means to Address the Problems

A cerebral aneurysm stent of the present invention comprises a hollow tubular main body and a coil support integrally formed with the main body, at a longitudinal end of the main body. The coil support includes a closed structure for a radial inner side of the cerebral aneurysm stent, at a longitudinal outer end of the cerebral aneurysm stent.

Further, according to an embodiment of the present invention, when the cerebral aneurysm stent is expanded, the coil support is expanded radially in a larger size than the main body, and the coil support is disposed in a cerebral aneurysm to support a coil inserted in the cerebral aneurysm. When the cerebral aneurysm stent is expanded, the closed structure of the coil support closes the radial inner side of the cerebral aneurysm stent to prevent escape of the coil.

Further, according to an embodiment of the present invention, the cerebral aneurysm stent further comprises a marker formed of a radiopaque material and disposed at tips of the closed structure.

Further, according to an embodiment of the present invention, the closed structure is a wire structure processed in to a linear shape different from a pattern of another portion of the coil support.

Further, according to an embodiment of the present invention, the marker is formed to tie or weld the tips of the closed structure.

Further, according to an embodiment of the present invention, when the cerebral aneurysm stent is expanded, the marker is positioned in a center of the radial inner side of the coil support.

Further, according to an embodiment of the present invention, the main body and the coil support are formed by weaving a shape memory alloy wire or by laser-cutting a shape memory alloy tube.

Meanwhile, a method for manufacturing a cerebral aneurysm stent of the present invention comprises the step a) of forming a hollow tubular main body and a coil support integrally formed with the main body at a longitudinal end of the main body; the step b) of forming a closed structure for a radial inner side of the cerebral aneurysm stent, at an outer end of the coil support in a radial direction of the cerebral aneurysm stent; the step c) of inserting the main body and the coil support into a jig; and the step d) expanding the main body and the coil support into a final shape by heat-treating the main body and the coil support in the jig.

Further, according to an embodiment of the present invention, in the step d), the coil support is expanded into a final shape radially larger than the main body, and wherein the closed structure is deformed to be flat to maintain a state of closing the radial inner side of the cerebral aneurysm stent.

Further, according to an embodiment of the present invention, in the step b), a marker formed of a radiopaque material is further disposed at tips of the closed structure.

Further, according to an embodiment of the present invention, in the step b), the closed structure is processed into a wire structure having a linear shape different from a pattern of another portion of the coil support.

Further, according to an embodiment of the present invention, in the step b), the marker is disposed by tying or welding the tips of the closed structure.

Further, according to an embodiment of the present invention, in the step d), the marker is positioned in a center of the radial inner side of the coil support.

Further, according to an embodiment of the present invention, in the step a) the main body and the coil support are formed by weaving a shape memory alloy wire or by laser-cutting a shape memory alloy tube.

### Effects of the Invention

According to the present invention, it is possible to address issues with the conventional Y-shaped overlapping stents used in stent-assisted coil embolization.

Specifically, it is possible to further simplify the stent-assisted coil embolization procedure than by Y-shaped overlapping stents. Further, since the cerebral aneurysm stent of the present invention has a tubular shape unlike the conventional Y-shaped overlapping stents, it is possible to overcome the risk of complications due to an overlap and obstacles to expansion of the stent.

Further, the cerebral aneurysm stent of the present invention may effectively prevent escape of the coil and easily realize adjustment of the installation position and the recovery of the stent.

Additionally, according to a method for manufacturing a cerebral aneurysm stent of the present invention, it is possible to manufacture a cerebral aneurysm stent, which solves the conventional problems, with high efficiency and convenience.

### BRIEF DESCRIPTION OF THE DRAWINGS

Fig. 1 illustrates a T-shaped branching vessel of a cerebral blood vessel in which a cerebral aneurysm mainly occurs.
FIG. 2 illustrates a state in which a cerebral aneurysm is filled with a coil in cerebral aneurysm coil embolization.
Fig. 3 illustrates a state in which conventional Y-shaped overlapping stents used for stent-assisted cerebral aneurysm coil embolization are disposed in T-shaped branching vessels of a cerebral blood vessel.
Fig. 4 illustrates a cerebral aneurysm stent according to an embodiment of the present invention.
Fig. 5 illustrates a state in which a cerebral aneurysm stent according to an embodiment of the present invention is disposed in T-shaped branching vessels of a cerebral blood vessel.
Fig. 6 specifically illustrates a method for manufacturing a cerebral aneurysm stent according to an embodiment of the present invention.
Fig. 7 specifically illustrates a method for manufacturing a cerebral aneurysm stent according to another embodiment of the present invention.
Fig. 8 is a flowchart illustrating a method for manufacturing a cerebral aneurysm stent according to an embodiment of the present invention.

### Best Mode To Practice the Invention

Hereinafter, embodiments of the present invention are described in detail with reference to the accompanying drawings. Further, the present invention is not limited to the following embodiments and may be realized in any feasible combination as well as individual manners that may be recognized by one of ordinary skill in the art. Furthermore, the scope of the present invention is not limited by the preferred embodiments. Further, the embodiments are schematically illustrated in the drawings. Herein, like reference numbers in the drawings indicate like or functionally similar elements.

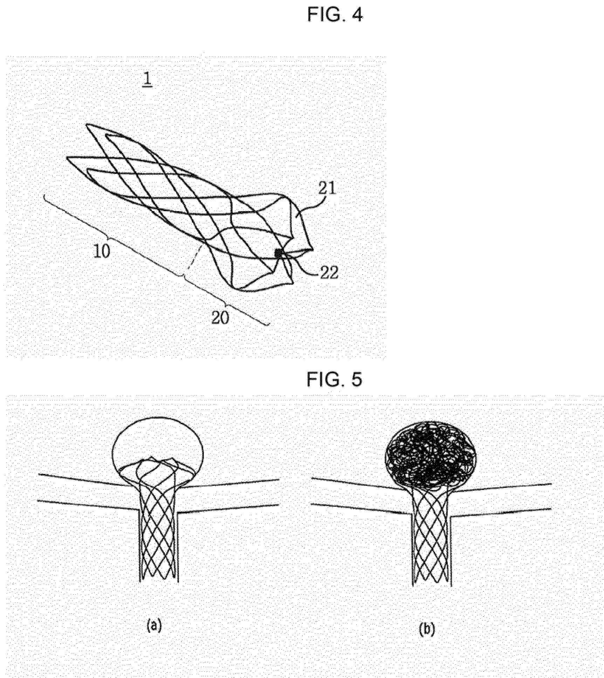

Fig. 4 illustrates a cerebral aneurysm stent according to an embodiment of the present invention. As illustrated in FIG. 4, the cerebral aneurysm stent 1 of the present invention includes a hollow tubular main body 10 and a coil support integrally formed with the main body 10 at one longitudinal end of the main body 10.

The coil support 20 includes, at the longitudinal outer end of the cerebral aneurysm stent 1, a closed structure 21 for the radial inner side of the cerebral aneurysm stent 1.

Due to the coil support 20 and the closed structure 21, the cerebral aneurysm stent 1 of the present invention may effectively prevent the coil from escaping from the cerebral aneurysm when placed in the T-shaped branching vessels of the cerebral vessel as shown in Fig. 5(b).

Specifically, if the longitudinal end of the stent is open, the coil may escape from the cerebral aneurysm due to the hollow shape of the stent. However, unlike a general stent, the coil support 20 of the cerebral aneurysm stent 1 of the present invention has the closed structure 21 for the radial inner side of the cerebral aneurysm stent 1, at the longitudinal end of the cerebral aneurysm stent 1, and when placed in the T-shaped branching vessels of the cerebral vessel as illustrated in FIG. 5(b), the closed structure 21 closes the radial inner side of the cerebral aneurysm stent 1, in other words, the closed structure 21 blocks the space where the coil escapes from the cerebral aneurysm, thus effectively preventing the coil from escaping from the cerebral aneurysm.

Meanwhile, the coil support 20 of the cerebral aneurysm stent 1 of the present invention is moved to the lesion site by a stent transfer device (not shown) to be placed inside the cerebral aneurysm. The main body 10 of the cerebral aneurysm stent 1 of the present invention expands to a size approximately corresponding to the entrance of the cerebral aneurysm when the cerebral aneurysm stent 1 is discharged from the stent transfer device. Further, the coil support 20 which is disposed inside the cerebral aneurysm is radially expanded in a larger size than the main body 10 when discharged from the stent transfer device as illustrated in FIG. 5(a).

Accordingly, as the coil support 20 of the cerebral aneurysm stent 1 supports a wider area, it is possible to further effectively prevent the coil inserted in the cerebral aneurysm from escaping off the cerebral aneurysm.

In other words, since the cerebral aneurysm stent 1 of the present invention has a hollow tubular main body 10 unlike the conventional Y-shaped overlapping stents used for stent-assisted coil embolization, it is possible to avoid the risk of complications and problems with stent expansion that the Y-shaped overlapped stents have. Further, unlike the Y-shaped overlapping stents, the cerebral aneurysm stent 1 of the present invention may facilitate the stent-assisted coil embolization procedure and adjustment of the position of installation or the recovery of the stent.

Moreover, unlike the Y-shaped overlapping stents, the cerebral aneurysm stent 1 of the present invention includes the coil support 20 integrally formed with the hollow tubular main body 10, at one longitudinal end of the main body 10, and the closed structure 21 for the radial inner side of the cerebral aneurysm stent 1 is included at the longitudinal outer end of the coil support 20. Thus, it is possible to effectively prevent the coil from escaping from the cerebral aneurysm. Further, the method for manufacturing the cerebral aneurysm stent of the present invention may manufacture the cerebral aneurysm stent, which address the conventional issues, with high efficiency and convenience.

Further, since the main body 10 and the coil support 20 are integrated with each other in the cerebral aneurysm stent 1 of the present invention, the cerebral aneurysm stent 1 may be manufactured with high efficiency and convenience as compared with when a main body and a coil support are formed as separate members.

Meanwhile, according to an embodiment of the present invention, as illustrated in FIG. 4, the cerebral aneurysm stent 1 may further include a marker 22 of radiopaque material at the tips of the closed structure 21.

The marker 22 may be formed of a platinum-iridium (Pt-Ir) alloy but, without being limited thereto, the marker 22 may be formed of another material having radiopacity. Due to the radiopacity of the marker 22, even after placed in the lesion site, the location of the stent may be easily identified through radiography.

Further, in the cerebral aneurysm stent 1 of the present invention, since the marker 22 is disposed at the tips of the closed structure 21, it is possible to reliably form the closed structure 21 using the marker 22.

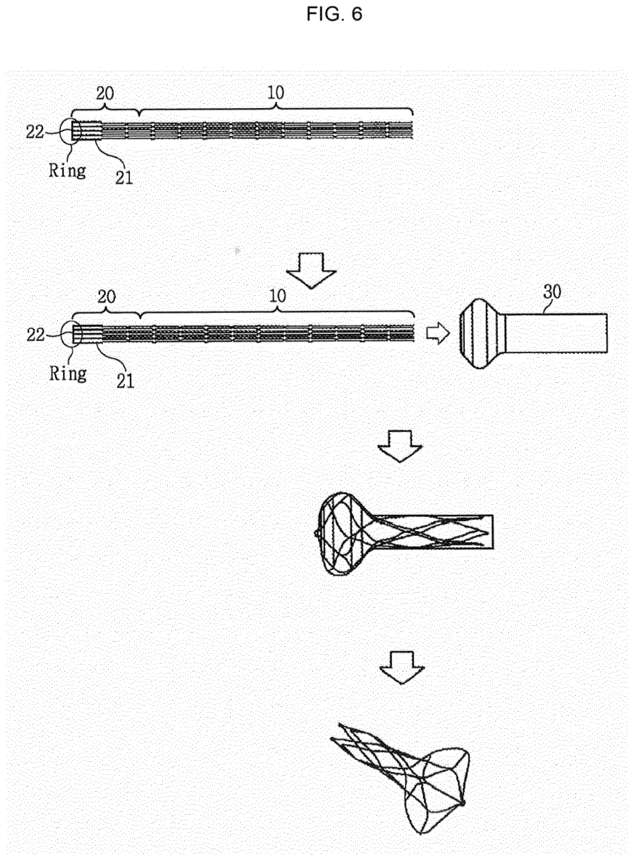

Specifically, as illustrated in FIG. 6, the closed structure 21 of the cerebral aneurysm stent 1 according to an embodiment of the present invention is formed into a wire structure processed into a linear shape different from the pattern of other portions of the coil support. The marker 22 in a ring shape is formed at the tips of the closed structural 21, and the tips of the closed structural 21 may be tied up with the marker 22.

Accordingly, as illustrated in FIGS. 4 and 6, since the closed structure 21 may reliably close the radial inner side of the cerebral aneurysm stent, it is possible to prevent escape of the coil.

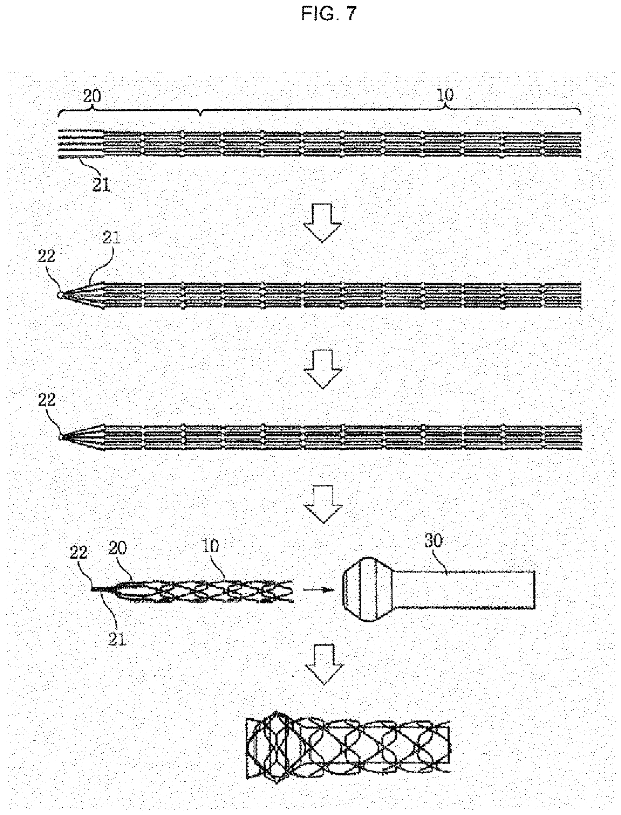

Alternatively, according to another embodiment of the present invention, as illustrated in FIG. 7, after collecting the tips of the closed structure 21, the tips of the closed structure 21 may be welded using the marker 22.

Further, if the marker 22 is formed at the tips of the closed structure 21 in such a manner, when the cerebral aneurysm stent 1 of the present invention is expanded, the marker 22 is positioned in the center of the radial inner side of the coil support 20. Thus, it may easily be identified whether the cerebral aneurysm stent 1 is supporting the coil in a balanced way.

The main body 10 and the coil support 20 of the cerebral aneurysm stent 1 of the present invention having the above characteristics are formed of, e.g., a shape memory alloy, such as NiTinol. Further, the cerebral aneurysm stent 1 of the present invention may be formed by weaving wires or by laser cutting a tube formed of a shape memory alloy, but is not limited thereto.

Next, a method for manufacturing a cerebral aneurysm stent 1 of the present invention is described with reference to FIGS. 6 to 8.

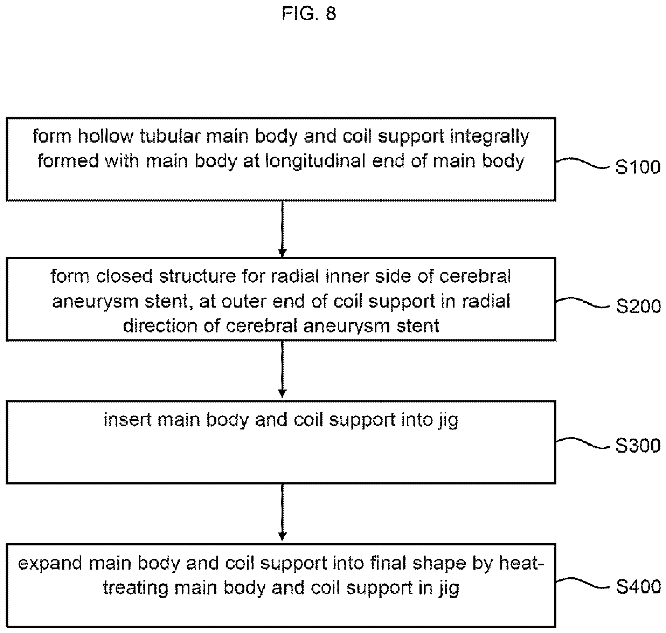

Referring to Fig. 8, a method for manufacturing a cerebral aneurysm stent 1 of the present invention includes the step S100 of forming a hollow tubular main body 10 and a coil support 20 integrally formed with the main body 10, at a longitudinal end of the main body 10; the step S200 of forming a closed structure 21 for a radial inner side of the cerebral aneurysm stent 10, at an outer end of the coil support 20 in a longitudinal direction of the cerebral aneurysm stent 1; the step S300 of inserting the main body 10 and the coil support 20 in a jig 30; and the step S400 of expanding the main body 10 and the coil support 20 into a final shape by heat-treating the main body 10 and the coil support 20 in the jig 30. Specifically, in step S100, the main body 10 and the coil support 20 may be formed by weaving shape memory alloy wires or by laser cutting a shape memory alloy tube.

If the main body 10 and the coil support 20 are integrally formed as described above, it is possible to manufacture with higher efficiency and convenience as compared with when the main body 10 and the coil support 20 are formed as separate members. Subsequently, in step S200, a closed structure 21 may be formed at the outer end of the coil support 20 in the longitudinal direction of the cerebral aneurysm stent 1. As illustrated in Figs. 6 and 7, the closed structure 21 is processed into a linear wire structure different from the pattern of other portions of the coil support 20.

Meanwhile, according to an embodiment of the present invention, a marker 22 of a radiopaque material may be additionally disposed at the tips of the closed structure 21 in step S200.

Further, as illustrated in FIG. 6, the marker 22 may be formed in a ring shape at the tips of the closed structure 21 so as to tie the tips of the closed structure 21.

Further, as illustrated in FIG. 7, after collecting the tips of the closed structure 21, the tips of the closed structure 21 may be welded using the marker 22 in step S200.

Then, in step S300, the main body 10 and the coil support 20 are inserted into a jig 30. The jig 30 has an internal space corresponding to the final shape of the cerebral aneurysm stent 1 of the present invention. For example, as illustrated in FIGS. 6 and 7, the jig 30 has an internal space to allow the final shape to be formed so that the coil support 20 has a larger radial size than the main body 10.

Subsequently, in step S400, heat treatment is performed on the main body 10 and the coil support 20 inserted in the jig 30 to expand the main body 10 and the coil support 20 into a final shape.

Since the cerebral aneurysm stent 1 is formed of a shape memory alloy, if the cerebral aneurysm stent 1 is processed into the final shape through heat treatment in step S400, the cerebral aneurysm stent 1 may return to its memorized final shape even when it undergoes deformation, such as crushing.

The heat treatment in step S400 may be performed by applying heat at 400 °C to 600 °C, preferably, 500 °C, for 15 minutes to 20 minutes. However, it should be understood that the heat treatment conditions are not limited thereto and may be appropriately adjusted according to the material and shape of the cerebral aneurysm stent 1.

Further, according to an embodiment of the present invention, since the jig 30 has an internal space that allows the coil support 20 to have a larger final shape in the radial direction than the main body 10, and the main body 10 and the coil support 20 are heattreated in the jig 30, the coil support 20 is expanded into the final shape larger in the radial direction than the main body 10.

Further, the closed structure 21 is deformed to be flat so as to keep the radial inner side of the cerebral aneurysm stent in the closed state as illustrated in FIGS. 6 and 7.

Further, when the marker 22 is disposed at the tips of the closed structure 21, the marker 22 is disposed in the center of the radial inner side of the coil support in the expanded cerebral aneurysm stent 1.

According to the method for manufacturing the cerebral aneurysm stent 1 of the present invention as described above, it is possible to manufacture, with high efficiency and convenience, the cerebral aneurysm stent 1 of the present invention which may address the above-described issues with the conventional Y-shaped overlapping stents used for stent-assisted coil embolization.

### [Legend of symbols]

1: cerebral aneurysm stent 10: main body
20: coil support 21: closed structure
22: marker 30: jig

## Claims

1. A cerebral aneurysm stent, comprising a hollow tubular main body and a coil support integrally formed with the main body, at a longitudinal end of the main body, wherein the coil support includes a closed structure for a radial inner side of the cerebral aneurysm stent, at a longitudinal outer end of the cerebral aneurysm stent.

2. The cerebral aneurysm stent of claim 1, wherein when the cerebral aneurysm stent is expanded, the coil support is expanded radially in a larger size than the main body, and the coil support is disposed in a cerebral aneurysm to support a coil inserted in the cerebral aneurysm, and wherein when the cerebral aneurysm stent is expanded, the closed structure of the coil support closes the radial inner side of the cerebral aneurysm stent to prevent escape of the coil.

3. The cerebral aneurysm stent of claim 1 or 2, further comprising a marker formed of a radiopaque material and disposed at tips of the closed structure.

4. The cerebral aneurysm stent of claim 1 or 2, wherein the closed structure is a wire structure processed in to a linear shape different from a pattern of another portion of the coil support.

5. The cerebral aneurysm stent of claim 3, wherein the marker is formed to tie or weld the tips of the closed structure.

6. The cerebral aneurysm stent of claim 3, wherein when the cerebral aneurysm stent is expanded, the marker is positioned in a center of the radial inner side of the coil support.

7. The cerebral aneurysm stent of claim 1 or 2, wherein the main body and the coil support are formed by weaving a shape memory alloy wire or by laser-cutting a shape memory alloy tube.

8. A method for manufacturing a cerebral aneurysm stent, the method comprising:
the step a) of forming a hollow tubular main body and a coil support integrally formed with the main body at a longitudinal end of the main body;
the step b) of forming a closed structure for a radial inner side of the cerebral aneurysm stent, at an outer end of the coil support in a radial direction of the cerebral aneurysm stent;
the step c) of inserting the main body and the coil support into a jig; and
the step d) expanding the main body and the coil support into a final shape by heat-treating the main body and the coil support in the jig.

9. The method of claim 8, wherein in the step d), the coil support is expanded into a final shape radially larger than the main body, and wherein the closed structure is deformed to be flat to maintain a state of closing the radial inner side of the cerebral aneurysm stent.

10. The method of claim 8 or 9, wherein in the step b), a marker formed of a radiopaque material is further disposed at tips of the closed structure.

11. The method of claim 8 or 9, wherein in the step b), the closed structure is processed into a wire structure having a linear shape different from a pattern of another portion of the coil support.

12. The method of claim 10, wherein in the step b), the marker is disposed by tying or welding the tips of the closed structure.

13. The method of claim 10, wherein in the step d), the marker is positioned in a center of the radial inner side of the coil support.

14. The method of claim 8 or 9, wherein in the step a), the main body and the coil support are formed by weaving a shape memory alloy wire or by laser-cutting a shape memory alloy tube.
